# FASCICULE DE BREVET EUROPEEN

(11) **EP 2 624 956 B1**
(45) Date de publication et mention de la délivrance du brevet: **14.08.2019**
(21) Numéro de dépôt: 11779791.0
(22) Date de dépôt: 04.10.2011
(51) Int. Cl.: B01L 3/00, G01N 33/49, G01N 35/04

(54) **CUVETTE DE RÉACTION POUR APPAREIL AUTOMATIQUE D'ANALYSE CHIMIQUE OU BIOLOGIQUE**
REAKTIONSGEFÄSS FÜR EIN AUTOMATISCHES CHEMISCHES ODER BIOLOGISCHES ANALYSEGERÄT
REACTION VESSEL FOR AN AUTOMATIC CHEMICAL OR BIOLOGICAL ANALYSIS DEVICE

(30) Priorité: 31.05.2011 FR 1154790; 05.10.2010 FR 1058041
(43) Date de publication de la demande: 14.08.2013
(73) Titulaire: Diagnostica Stago, 92600 Asnières-sur- Seine (FR)
(72) Inventeur: CROISARD, Philippe, F-78260 Acheres (FR); VALVERDE, Olivier, F-92300 Levallois (FR)
(74) Mandataire: Pichat, Thierry
(86) Numéro de dépôt international: PCT/FR2011/052310
(87) Numéro de publication internationale: WO 2012/045972

(56) Documents cités:
- EP-A1- 0 325 874
- EP-A1- 1 792 656
- EP-A2- 1 678 509
- WO-A1-03/065047
- WO-A2-2007/039524
- FR-A1- 2 873 447
- FR-A1- 2 896 589
- FR-A1- 2 917 828
- US-A1- 2008 317 641

## Description

L'invention concerne une cuvette de réaction pour appareil automatique d'analyse chimique ou biologique, ainsi que des ensembles formés de ces cuvettes et des dispositifs de préhension et d'assemblage de ces cuvettes.

On connaît par les documents EP-A-325874 et WO-A-03/065047 des cuvettes de ce type qui sont utilisées par exemple pour la détermination des temps de modification de l'état physique d'un milieu, en particulier pour la détermination du temps de coagulation d'un échantillon de sang, ces cuvettes étant ouvertes à leur extrémité supérieure et comportant un fond incurvé formant un chemin de roulement d'une bille de matériau ferromagnétique qui est déplaçable dans la cuvette en mouvement périodique au moyen d'un champ magnétique extérieur, les variations d'amplitude et/ou de fréquence des déplacements de la bille étant représentatives de l'état physique de l'échantillon de sang.

Dans le document WO-A-03/065047, les cuvettes contenant les billes sont fixées côte à côte de façon détachable sur un film support souple qui obture leurs extrémités supérieures et qui peut être enroulé sur une bobine pour alimenter un appareil automatique d'analyse et faire défiler les cuvettes en succession dans l'appareil.

Le film comporte des fentes ou orifices au droit des ouvertures des cuvettes, pour le dépôt d'échantillons et de réactifs dans les cuvettes, tout en maintenant les billes dans les cuvettes.

A leur extrémité supérieure, les cuvettes comportent deux rebords latéraux ou oreilles formés avec des picots en saillie destinés à être engagés à force dans des perforations latérales du film, les rebords des cuvettes fixés au film formant une crémaillère permettant de déplacer l'ensemble du film et des cuvettes par engrènement avec une courroie crantée ou analogue.

Ces moyens connus présentent de nombreux avantages mais aussi quelques inconvénients : il faut fixer les cuvettes sur le film, puis les en détacher après utilisation, ces opérations pouvant ne pas être exécutées de façon parfaite, avec des risques de détachement prématuré ou tardif des cuvettes. Elles ont également un coût, qui n'est pas négligeable par rapport à celui des cuvettes et qui s'ajoutent à celui du film auquel sont fixées les cuvettes.

Le document US2008317641 A1 dévoile une chaîne de tubes à essais. Sur chacun d'entre eux vient se fixer une pièce de liaison qui permet d'assembler plusieurs tubes les uns aux autres.

Dans le document EP1792656 A1, une seule pièce de liaison vient relier plusieurs cuvettes entre elles.

La référence FR2896589 A1 décrit une cuvette de réaction comprenant une languette destinée à venir coiffer l'encoche d'une cuvette adjacente. Une bille ferromagnétique sert à agiter le fluide présent dans la cuvette.

La cuve du document FR2917828 A1 comporte au moins un premier volet d'obturation monté sur la paroi latérale de ladite cuve, afin de fermer le volume de réception et retenir une bille ferromagnétique.

Il peut arriver aussi, au cours des déplacements des cuvettes ou des bobines sur lesquelles sont enroulés les films portant les cuvettes, que les billes contenues dans des cuvettes ne soient pas bien retenues par le film portant les cuvettes et soient éjectées des cuvettes. Dans ce cas, les analyses prévues ne peuvent être effectuées correctement dans les cuvettes et doivent être refaites, ce qui se traduit par une perte d'échantillons, de réactifs et de temps.

De plus, le chargement des cuvettes en bobine dans l'appareil d'analyse ne permet pas de disposer d'un stock tampon de cuvettes dans l'appareil au moment du changement de bobine, ce qui impose l'arrêt de l'appareil pendant ce changement. Le dessertissage du film et des cuvettes peut aussi entraîner des ruptures des picots ou des rebords latéraux des cuvettes, ainsi que des sauts de billes, ce qui peut entraîner un blocage de l'appareil ou du dispositif de chargement des cuvettes.

La présente invention a notamment pour but d'éviter ces inconvénients de la technique connue, d'une façon simple, efficace et peu coûteuse.

Elle propose à cet effet une cuvette de réaction pour appareil automatique d'analyse chimique ou biologique selon la revendication indépendante 1.

Ainsi, selon l'invention, les cuvettes sont reliées entre elles par des pièces de liaison dont chacune est portée par l'extrémité supérieure d'une cuvette et est accrochée sur la pièce de liaison d'une autre cuvette. Cette liaison des cuvettes évite les inconvénients liés à l'utilisation du film plastique dans la technique antérieure et permet aussi de supprimer les picots prévus sur les rebords latéraux des cuvettes dans la technique antérieure.

Avantageusement, la pièce de liaison comporte des moyens d'accrochage sur la pièce de liaison d'une autre cuvette et d'articulation autour d'un axe transversal parallèle à un bord de l'extrémité supérieure de la cuvette.

Ainsi, les cuvettes reliées entre elles forment une suite articulée, ce qui facilite le stockage et le rangement des cuvettes, ainsi que leur chargement et leur déplacement dans un appareil automatique d'analyse.

Selon une autre caractéristique avantageuse de l'invention, les moyens d'accrochage de la pièce de liaison sont des moyens de clipsage ou d'encliquetage élastique.

Grâce à cette caractéristique, les pièces de liaison peuvent être montées sur les cuvettes et accrochées les unes aux autres sans qu'il soit nécessaire d'utiliser un outil quelconque.

Selon l'invention, la pièce de liaison fixée sur l'extrémité supérieure de la cuvette comprend des moyens de retenue du moyen d'agitation de fluide, ces moyens de retenue s'étendant à l'intérieur de la cuvette.

Selon l'invention, le moyen d'agitation de fluide contenu dans la cuvette est une bille de matériau ferromagnétique, les moyens de retenue formés sur la pièce de liaison comprennent au moins une plaque mince s'étendant vers le bas à l'intérieur de la cuvette le long d'une paroi longitudinale de celle-ci et présentant un bord inférieur formant une arête de guidage et de retenue de la bille précitée.

De préférence, la pièce de liaison comprend deux plaques minces précitées qui sont parallèles et s'étendent vers le bas le long de deux parois longitudinales opposées de la cuvette et dont les bords inférieurs forment deux arêtes parallèles de guidage et de retenue de la bille ferromagnétique dans la cuvette.

Les bords verticaux de la plaque mince précitée ou de chaque plaque mince peuvent être guidés entre la paroi longitudinale de la cuvette et des nervures verticales formées en saillie sur les faces internes des parois transversales de la cuvette.

Selon une variante de réalisation de l'invention, la pièce de liaison comporte une languette refermant, au moins en partie, l'extrémité supérieure ouverte de la cuvette de manière à retenir le moyen d'agitation du fluide dans la cuvette.

De manière préférée, la languette est élastique et peut être repliée vers l'intérieur de la cuvette pour permettre un accès plus facile au volume interne de la cuvette et le dépôt par exemple d'un échantillon ou d'un réactif.

Selon une autre variante de réalisation, la pièce de liaison comporte une languette s'étendant vers l'extérieur et destinée à refermer, au moins en partie, l'extrémité supérieure ouverte d'une cuvette adjacente à laquelle ladite cuvette est accrochée, de manière à retenir le moyen d'agitation du fluide dans la cuvette adjacente.

De cette manière, une fois les cuvettes accrochées l'une à l'autre, la languette d'une cuvette empêche le moyen d'agitation de sortir accidentellement d'une cuvette adjacente.

Dans un mode de réalisation préféré de l'invention, la pièce de liaison comprend un cadre rectangulaire qui coiffe l'extrémité supérieure ouverte de la cuvette et qui porte des moyens d'accrochage sur une pièce de liaison d'une autre cuvette.

Ce cadre rectangulaire de la pièce de liaison comprend deux bras parallèles s'étendant vers l'extérieur dans le prolongement de ses petits côtés, et les extrémités de ces deux bras comportent des moyens d'accrochage sur des moyens complémentaires formés sur le cadre rectangulaire d'une pièce de liaison d'une autre cuvette.

Le cadre rectangulaire de la pièce de liaison comporte également des moyens de clipsage ou d'encliquetage élastique sur l'extrémité supérieure de la cuvette.

Avantageusement, la pièce de liaison précitée est en matériau plastique sensiblement opaque.

Ainsi, le contenu de la cuvette de réaction est protégé contre un éclairage par une source de lumière extérieure, ce qui dans certains cas facilite ou améliore la lecture d'un résultat de réaction.

L'invention propose également un ensemble de cuvettes de réaction pour un appareil automatique d'analyse chimique ou biologique, caractérisé en ce que cet ensemble comprend une pluralité de cuvettes de réaction du type décrit ci-dessus, qui sont reliées les unes aux autres en file continue par les pièces de liaison et qui sont enroulées en spirale sur un support circulaire de chargement dans l'appareil d'analyse ou qui sont disposées en rangées parallèles dans un chargeur vertical ou horizontal.

L'invention concerne également un ensemble comportant au moins une cuvette de réaction et un dispositif de préhension de ladite cuvette de réaction, caractérisée en ce que le dispositif de préhension comprend une tige cylindrique mobile en déplacement vertical et portant des moyens d'accrochage sur le cadre rectangulaire de la pièce de liaison, ces moyens d'accrochage étant déplaçables en translation horizontale sur la tige cylindrique.

L'invention propose enfin un procédé d'assemblage de cuvettes de réaction du type décrit ci-dessus selon la revendication indépendante 14, comprenant les étapes consistant à :
- déposer des cuvettes sur des supports comportant des emplacements de réception et de positionnement des cuvettes, ces emplacements étant agencés pour former des files de cuvettes espacées les unes des autres d'un pas prédéterminé ;
- déposer un moyen d'agitation, tel par exemple qu'une bille ferromagnétique, dans chacune des cuvettes positionnées sur les supports précités ;
- déposer et encliqueter élastiquement des pièces de liaison sur les cuvettes positionnées sur les supports précités ;
- vérifier par contrôle vidéo la présence des moyens d'agitation dans les cuvettes et l'encliquetage des pièces de liaison sur les cuvettes ;
- déplacer les supports de cuvettes dans différents postes conçus pour effectuer les étapes précitées.

Ce dispositif d'assemblage des cuvettes est beaucoup plus simple et fiable que le dispositif d'assemblage à film plastique de la technique antérieure.

L'invention sera mieux comprise et d'autres caractéristiques, détails et avantages de celle-ci apparaîtront plus clairement à la lecture de la description qui suit, faite à titre d'exemple en référence aux dessins annexés, dans lesquels :
- les figures 1 et 2 sont des vues schématiques en perspective et en coupe d'une cuvette de réaction selon l'invention ;
- la figure 3 est une vue schématique en perspective d'une pièce de liaison ;
- la figure 4 est une vue schématique en perspective représentant une cuvette équipée d'une pièce de liaison ;
- la figure 5 est une vue schématique en perspective représentant plusieurs cuvettes reliées par des pièces de liaison ;
- la figure 6 est une vue schématique en perspective d'une cuvette équipée d'une pièce de liaison selon une variante de réalisation de l'invention,
- les figures 7 et 8 sont des vues schématiques en perspective, respectivement de dessus et de dessous, de la pièce de liaison de la figure 6,
- la figure 9 est une vue schématique en perspective représentant plusieurs cuvettes accrochées les unes aux autres par des pièces de liaison, selon une autre variante de réalisation de l'invention,
- la figure 10 est une vue en perspective et de dessus, d'une cuvette et d'une pièce de liaison selon la variante de la figure 9,
- la figure 11 est une vue en perspective et de dessous, de la pièce de liaison de la figure 10,
- la figure 12 est une vue schématique en perspective d'un tiroir de rangement de cuvettes selon l'invention ;
- la figure 13 est une vue schématique en perspective d'un chargeur horizontal comportant des tiroirs du type de celui représenté en figure 12 ;
- la figure 14 est une vue schématique en perspective d'un chargeur vertical comportant des tiroirs du type de celui représenté en figure 12 ;
- la figure 15 est une vue schématique en perspective d'un dispositif de préhension d'une cuvette selon l'invention ;
- les figures 16a et 16b illustrent schématiquement le fonctionnement du dispositif de préhension de la figure 15 ;
- la figure 17 représente schématiquement un dispositif d'assemblage de cuvettes selon l'invention.

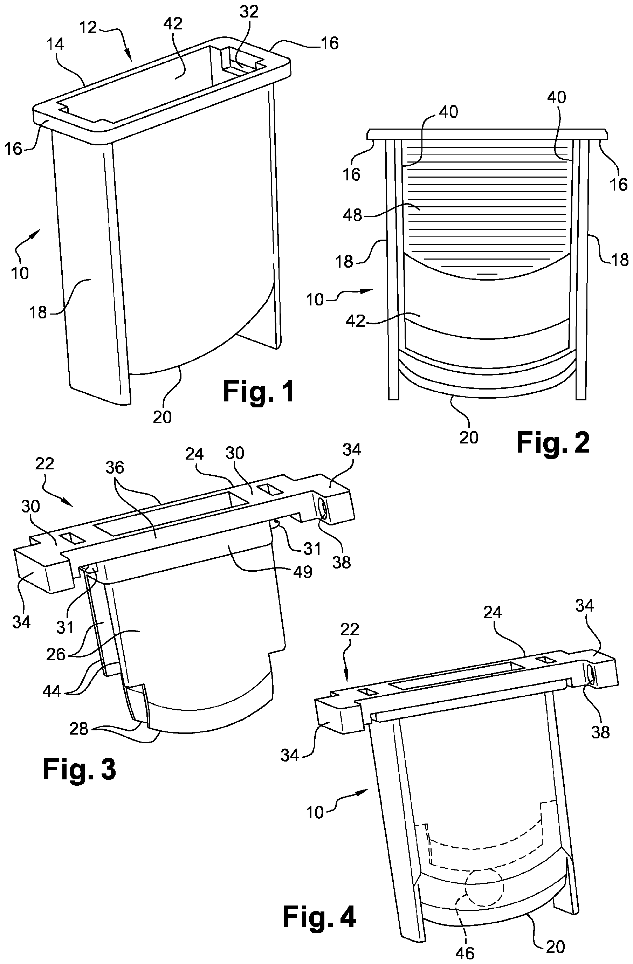

On se réfère d'abord aux figures 1 à 4 qui représentent un premier mode de réalisation de l'invention dans lequel une cuvette de réaction 10 réalisée en matériau plastique transparent est de forme générale parallélépipédique rectangle et comprend une extrémité supérieure ouverte 12 entourée d'un cadre 14 présentant deux rebords latéraux ou oreilles 16 raccordés aux petites parois latérales 18 de la cuvette et qui sont perpendiculaires à ces petites parois latérales.

Le fond 20 de la cuvette est incurvé à concavité tournée vers le haut et forme un chemin de guidage d'un moyen d'agitation de fluide constitué par une bille en matériau ferromagnétique telle que celle 46 représentée en figure 4.

Cette cuvette de réaction 10 a typiquement une hauteur de 15mm, et une section transversale de 10mm x 4mm, avec un volume utile de 490mm³ (420mm³ quand la cuvette est munie de la pièce de liaison).

Elle est associée à une pièce de liaison 22 représentée en figure 3, qui est réalisée en matériau plastique de préférence opaque et qui comprend essentiellement à son extrémité supérieure un cadre rectangulaire 24 et deux plaques minces ou lamelles 26 verticales, qui sont parallèles et raccordées à leur extrémité supérieure aux grands côtés du cadre 24, ce deux lamelles 26 étant identiques et l'image l'une de l'autre dans un miroir.

Leur extrémité inférieure 28 est incurvée à la fois dans un plan longitudinal et dans un plan transversal, d'une façon correspondant à la courbure du fond incurvé 28 de la cuvette 10, les deux extrémités inférieures 28 des lamelles 26 formant des arêtes de guidage et de retenue de la bille 46 de matériau ferromagnétique posée dans la cuvette 10 sur le fond incurvé de celle-ci. Ces extrémités 28 sont écartées l'une de l'autre d'une distance inférieure au diamètre de la bille.

Le cadre rectangulaire 24 a des dimensions sensiblement égales ou légèrement supérieures à celles du cadre 14 de la cuvette 10 et vient s'appliquer sur ce cadre 14 lorsque la pièce de liaison 22 est montée à l'intérieur de la cuvette 10 comme représenté schématiquement en figure 4.

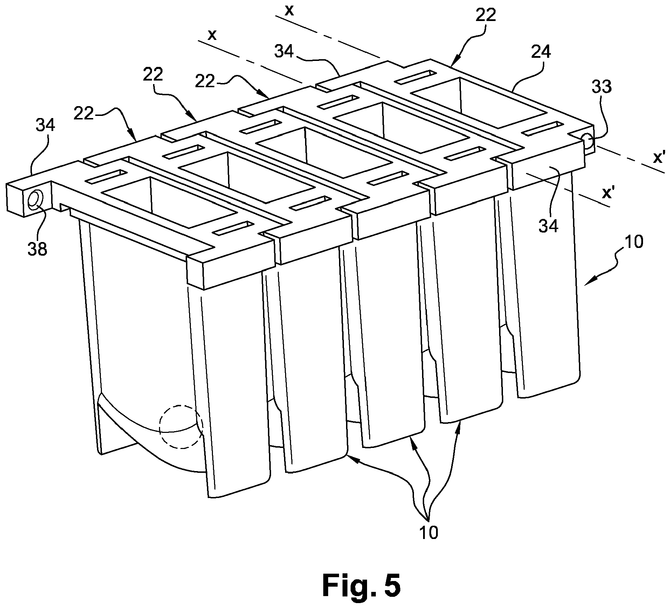

Les petits côtés 30 du cadre 24 comprennent, sur leur face inférieure, des rebords 31 d'accrochage par clipsage ou encliquetage élastique dans des fenêtres 32 des extrémités supérieures des petites parois de la cuvette, les extrémités des rebords 31 s'engageant sous les rebords ou oreilles 16 du cadre 14 de la cuvette. Les faces verticales extérieures de ces petits côtés du cadre comprennent également chacune une demi-sphère 33 en saillie (figure 5), les deux demi-sphères 33 étant alignées parallèlement à un grand côté du cadre 24 et faisant partie des moyens d'accrochage de la pièce de liaison 22 sur une autre pièce de liaison identique associée à une autre cuvette 10.

Le cadre 24 comprend de plus deux branches parallèles 34 formées aux extrémités d'un grand côté 36 du cadre 24, les branches 34 s'étendant perpendiculairement à ce grand côté 36 et étant séparées l'une de l'autre d'une distance égale à la longueur de l'autre grand côté du cadre 24. La face verticale interne de chaque branche 34 comporte une cavité semi sphérique 38 destinée à recevoir une saillie semi sphérique 33 d'une autre pièce de liaison 22 lorsque deux cuvettes 10 doivent être reliées l'une à l'autre comme représenté en figure 5, les deux cavités semi sphériques 38 des branches 34 étant alignées l'une avec l'autre parallèlement au grand côté 36 du cadre 24.

Par ailleurs, des moyens sont prévus pour que les lamelles 26 de la pièce 22 restent parallèles quand la pièce de liaison 22 est montée sur la cuvette 10, afin que les extrémités inférieures 28 des lamelles 26 puissent bien jouer leur rôle de guidage et de retenue de la bille ferromagnétique 46 reposant sur le fond incurvé 20 de la cuvette. Ces moyens sont par exemple constitués par des nervures verticales 40 formées en saillie sur les faces internes des parois latérales 18 de la cuvette, ces nervures 40 délimitant avec les grandes parois verticales 42 de la cuvette des glissières de guidage des bords verticaux 44 des lamelles 26 de la pièce 22.

Comme on l'a représenté schématiquement en figure 2, la face interne de chaque grande paroi 42 de la cuvette est « dépolie », c'est-à-dire n'est pas lisse et présente une certaine rugosité, au moins dans sa partie supérieure 48, pour limiter les remontées de liquide par capillarité dans le faible intervalle formé entre cette paroi et la lamelle 26 correspondante de la pièce de liaison. De plus, une bande horizontale 49 est formée en surépaisseur sur la face externe de chaque lamelle 26, à son extrémité supérieure, cette bande 49 étant appliquée sur la face interne de la paroi 42 de la cuvette quand la pièce 22 est montée sur la cuvette et jouant le rôle d'un joint ou d'un bourrelet d'étanchéité fermant l'intervalle entre la lamelle 26 et la paroi 42 de la cuvette.

Pour l'assemblage des cuvettes les unes aux autres, une pièce de liaison 22 est présentée au-dessus de chaque cuvette 10 contenant une bille ferromagnétique 46 et est descendue dans la cuvette jusqu'à ce que le cadre 24 de la pièce 22 repose sur le cadre 14 de la cuvette et soit fixé sur ce cadre par clipsage ou encliquetage élastique. Dans cette position, les extrémités inférieures 28 des lamelles 26 de la pièce 22 sont situées juste au-dessus de la bille de matériau ferromagnétique qui repose sur le fond incurvé 20 de la cuvette et empêchent cette bille de sortir de la cuvette. Il suffit ensuite d'accrocher les cuvettes les unes aux autres en engageant les branches 34 d'une pièce de liaison 22 d'une cuvette sur les petits côtés 30 du cadre 24 d'une autre pièce de liaison 22 fixée sur une autre cuvette, l'accrochage des deux pièces de liaison 22 l'une sur l'autre se faisant par clipsage ou encliquetage élastique grâce à une légère déformation vers l'extérieur des branches 34 lorsqu'elles sont engagées sur les saillies semi sphériques 32 de l'autre pièce de liaison. On peut ainsi accrocher les unes aux autres une série de cuvette 10 pour former une file comme représenté en figure 5, les cuvettes étant articulées les unes aux autres autour d'axes transversaux xx' formés par les saillies 33 logées dans les cavités 38.

Dans la file de cuvettes ainsi constituée, les branches 34 des pièces de liaison 22 forment des saillies d'engrènement avec des dents d'un organe de déplacement dans un appareil d'analyse automatique.

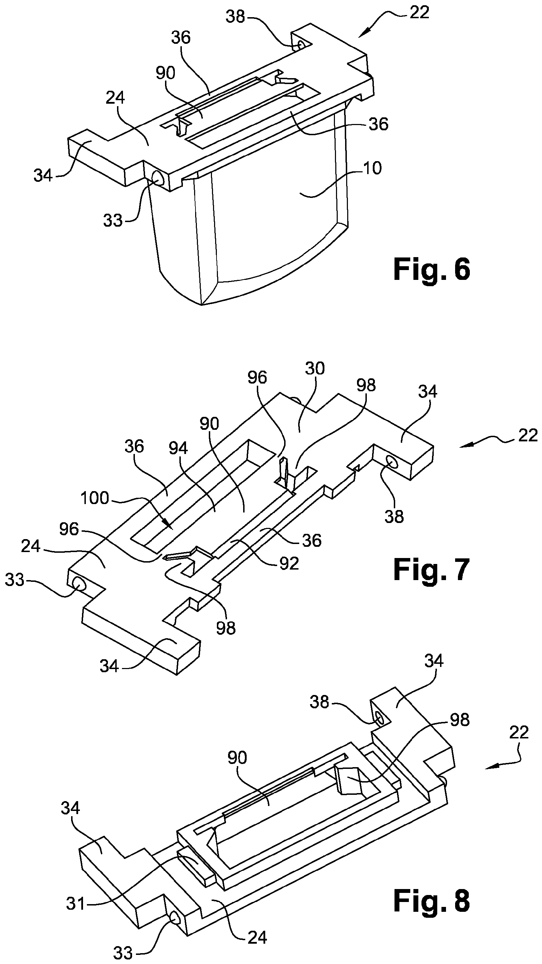

Les figures 6 à 8 illustrent une variante de réalisation de l'invention dans laquelle la pièce de liaison 22 est dépourvue des lamelles 26 verticales. Le maintien de la bille 46 à l'intérieur de la cuvette 10 est assuré par une languette ou un volet 90 refermant, au moins en partie, l'extrémité supérieure ouverte 12 de la cuvette 10, de manière à retenir la bille 46 dans la cuvette 10.

Ce volet 90 vient de moulage avec le cadre 24 et présente une forme générale trapézoïdale. Le volet 90 comporte un petit bord longitudinal 92 relié à l'un des grands côtés 36 du cadre 24, et un grand bord longitudinal 94 comprenant deux extrémités 96 reliées de manière sécable respectivement aux petits côtés 30 du cadre 24 (figure 7).

Après avoir sectionné les zones de liaison entre les extrémités précitées 96 du volet 90 et le cadre 24, par exemple par appui sur le volet 90, on peut faire pivoter ce dernier autour de son petit bord longitudinal 92, entre une position d'obturation, représentée aux figures 6 et 7, dans laquelle il s'étend dans le plan du cadre 24 et referme au moins en partie l'ouverture du cadre 24 et de la cuvette 10, et une position d'accès dans laquelle il est rabattu vers le bas de manière à permettre un accès plus facile au volume interne de la cuvette 10, afin d'y déposer par exemple un échantillon ou un réactif.

Chaque petit côté 30 du cadre 24 comporte en outre un ergot d'encliquetage 98 s'étendant, dans l'ouverture du cadre 24, en direction du petit côté 30 opposé. Les ergots 98 maintiennent le volet 90 en position d'accès.

Dans le cas représenté aux figures 6 à 8, le volet 90 ne ferme pas entièrement l'ouverture du cadre 24, une fente 100 d'une largeur inférieure au diamètre de la bille 46 étant ménagée entre le volet 90 et le grand côté 36 opposé du cadre 24. De cette manière, en position d'obturation, la bille 46 ne peut pas être extraite de la cuvette 10.

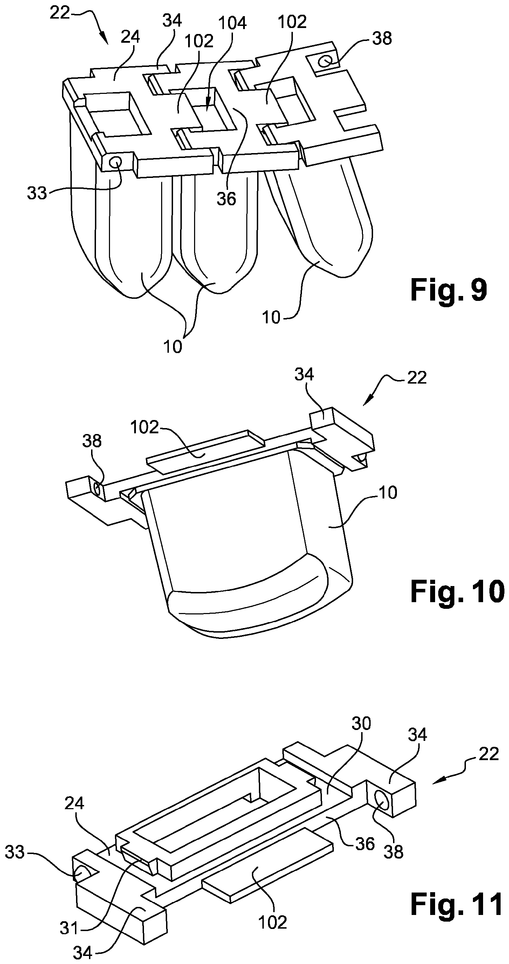

Les figures 9 à 11 illustrent une autre variante de réalisation dans laquelle la pièce de liaison 22 est également dépourvue des lamelles 26 verticales et le maintien de la bille 46 à l'intérieur de la cuvette 10 est assuré par une languette 102 d'une pièce de liaison adjacente 22.

Chaque pièce de liaison 22 comporte une languette 102 s'étendant vers l'extérieur depuis le grand côté 36 du cadre 24 auquel sont rattachés les rebords 34. La languette 102 s'étend dans le plan du cadre 24, sa longueur étant déterminée de façon à ce que ladite languette 102 ferme, au moins en partie, l'extrémité supérieure ouverte 12 d'une cuvette adjacente à laquelle ladite cuvette est accrochée, de manière à retenir la bille 46 dans la cuvette adjacente.

Dans le cas représenté aux figures 9 à 11, la languette 102 ne ferme pas entièrement l'ouverture 12 de la cuvette adjacente 10, une fente 104 (figure 9) d'une largeur inférieure au diamètre de la bille 46 étant ménagée entre l'extrémité libre de la languette 102 et le grand côté 36 opposé du cadre correspondant 24. De cette manière, la bille 46 ne peut pas être extraite de la cuvette adjacente 10.

Les pièces de liaison 22 des figures 6 à 11 restent similaires à celle décrite précédemment en référence aux figures 1 à 5 en ce qu'elle comportent également des branches 34 et des moyens d'accrochage 33, 38 destinés à coopérer avec des moyens d'accrochage complémentaires d'une pièce de liaison 22 d'une autre cuvette 10. Ces cuvettes 10 peuvent donc être accrochées les unes aux autres de manière à former une file de cuvettes 10.

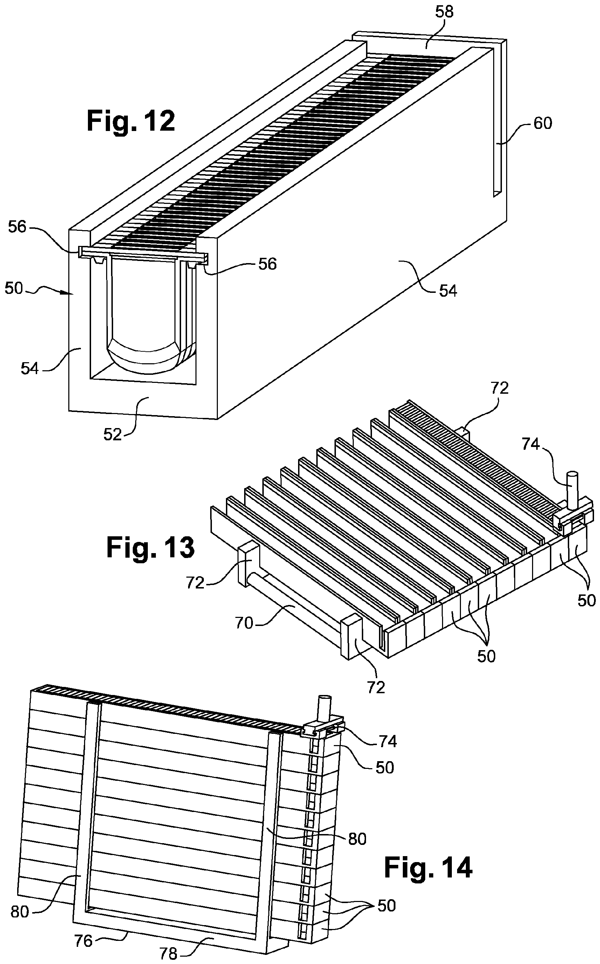

Les files de cuvettes de réaction peuvent être rangées dans des tiroirs 50 tels que celui représenté en figure 12, ce tiroir comprenant un fond 52 et deux parois latérales parallèles 54 réunies par le fond 52, ces deux parois latérales 54 comprenant au voisinage de leur extrémité supérieure une rainure 56 horizontale formant une glissière de guidage et de retenue des rebords 34 des pièces de liaison 22.

Une extrémité du tiroir 50 est fermée par une paroi transversale 58 perpendiculaire aux parois latérales 54, l'autre extrémité du tiroir étant ouverte pour permettre l'entrée et la sortie des cuvettes de réaction.

Eventuellement, au voisinage de la paroi 58, une fente 60 peut être pratiquée dans les parois 54 pour le passage d'un poussoir permettant de déplacer les cuvettes de réaction vers l'extrémité opposée ouverte du tiroir 50.

Les tiroirs 50 garnis de cuvettes de réaction peuvent être disposés dans un chargeur horizontal tel que celui représenté en figure 13 ou dans un chargeur vertical tel que celui de la figure 14.

Le chargeur de la figure 13 comprend un cadre horizontal 70 comprenant des rebords 72 de retenue des tiroirs 50 qui sont posés côte à côte sur ce cadre.

La référence 74 désigne un dispositif qui peut être utilisé dans l'appareil d'analyse pour pousser les cuvettes de réaction logées dans un tiroir 50 et les faire sortir de ce tiroir.

Le chargeur vertical 76 de la figure 14 comprend un fond 78 sur lequel on peut poser une pile de tiroirs 50 superposés verticalement, qui sont retenus sur le fond 78 par des montants verticaux 80 portés par ce fond.

Le même dispositif 74 qu'en figure 13 peut être utilisé pour sortir les cuvettes de réaction du tiroir supérieur 50.

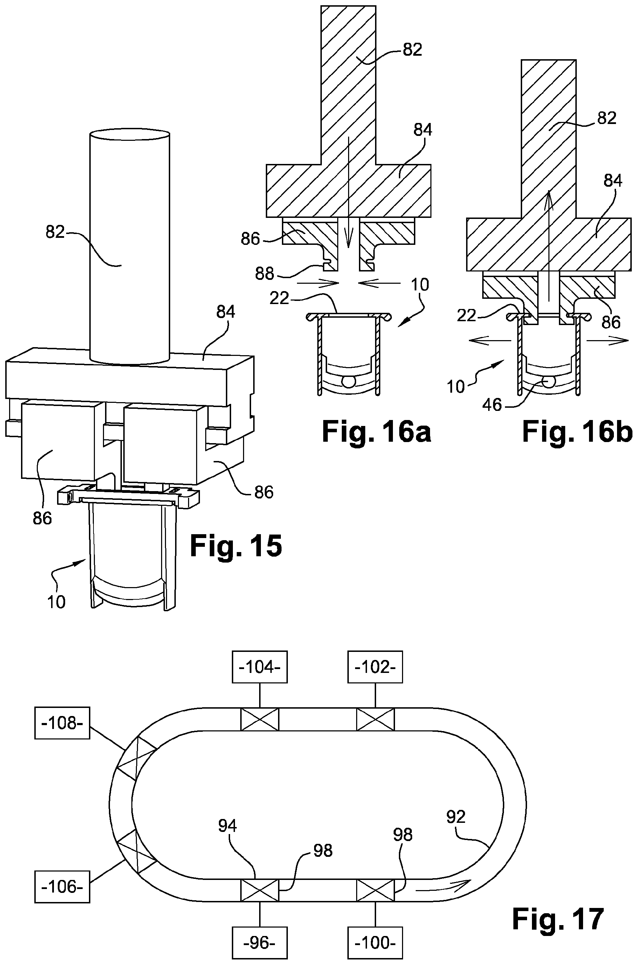

Les figures 15 et 16 représentent schématiquement un dispositif de préhension de cuvette dans un appareil automatique d'analyse, ce dispositif pouvant être utilisé avec les cuvettes de réaction équipées des pièces de liaison précitées.

Ce dispositif de préhension comprend une tige verticale cylindrique 82 dont l'extrémité inférieure porte une barre horizontale 84 sur laquelle deux doigts d'accrochage 86 sont guidés en translation horizontale, l'un vers l'autre et à l'opposé l'un de l'autre. Les doigts 86 portent à leur extrémité inférieure des becs d'accrochage 88 destinés à s'engager sur les petits côtés du cadre 24 d'une pièce de liaison montée sur une cuvette 10.

Le fonctionnement de ce dispositif de préhension est le suivant :
comme représenté en figure 16a, le dispositif est amené au-dessus d'une cuvette de réaction 10, les deux doigts 86 étant rapprochés l'un de l'autre sur la barre transversale 84 du dispositif. Par descente de la tige 82 comme représenté en figure 16b, on amène les becs d'accrochage 88 à l'intérieur de la cuvette 10, au niveau de la pièce de liaison 22, puis on déplace les deux doigts 86 à l'opposé l'un de l'autre pour engager les becs 88 sur les petits côtés du cadre de la pièce de liaison 22. Par déplacement vers le haut de la tige 82, on peut sortir la cuvette de réaction 10, par exemple de son logement dans l'appareil d'analyse et la déplacer vers un autre endroit.

On voit dans les figures 16a et 16b la bille 46 de matériau ferromagnétique qui est logée dans la cuvette de réaction et guidée sur le fond incurvé de celle-ci. On voit également les extrémités inférieures incurvées des lamelles de la pièce de liaison, qui empêchent cette bille de sortir de la cuvette de réaction lors des manipulations de cette dernière.

La figure 17 représente schématiquement un dispositif d'assemblage des cuvettes de réaction et des pièces de liaison.

Ce dispositif comprend un convoyeur 92 qui relie différents postes de chargement des cuvettes de réaction et des pièces de liaison et qui permet de déplacer des ensembles de cuvettes d'une extrémité à l'autre de ce dispositif, dans le sens indiqué par la flèche.

Plus précisément, ce dispositif comprend un poste 94 dans lequel des cuvettes contenues dans une unité de stockage 96 peuvent être déposées et positionnées sur des supports appropriés 98 portés par le convoyeur 92.

Les supports 98 comportent par exemple des logements de cuvettes agencés en rangées parallèles, de sorte que les cuvettes placées dans ces logements occupent des positions correspondant à celles qu'elles occuperont dans une file de cuvettes de la figure 5.

Le poste suivant 100 est un poste de dépôt de billes ferromagnétiques dans les cuvettes portées par le support 98.

Le poste suivant 102 est un poste de pose des pièces de liaison sur les cuvettes de réaction et de clipsage des pièces de liaison entre elles pour former des files de cuvettes de réaction.

Le poste suivant 104 est un poste de contrôle équipé d'une caméra vidéo pour une vérification de la présence des billes ferromagnétiques dans les cuvettes et du bon clipsage des pièces de liaison sur les cuvettes et des pièces de liaison entre elles.

Le dispositif comprend encore, en entrée, un poste 106 de pose des supports 98 vides sur le convoyeur 92 et, en sortie, un poste 108 de prise des supports 98 chargés de files de cuvettes de réaction.

## Revendications

1. Cuvette de réaction pour appareil automatique d'analyse chimique ou biologique, cette cuvette ayant une extrémité supérieure ouverte et contenant un moyen d'agitation d'un fluide, où l'extrémité supérieure de la cuvette (10) porte une pièce (22) de liaison, par accrochage, à une pièce de liaison identique (22) d'une autre cuvette (10) du même type, la pièce de liaison (22) étant fixée par clipsage ou encliquetage élastique sur l'extrémité supérieure de la cuvette, la pièce de liaison (22) comprenant des moyens (28) de retenue du moyen (46) d'agitation de fluide, ces moyens de retenue s'étendant à l'intérieur de la cuvette, le moyen d'agitation de fluide étant une bille (46) de matériau ferromagnétique, les moyens de retenue formés sur la pièce de liaison (22) comprennant au moins une plaque mince ou lamelle (26) s'étendant vers le bas à l'intérieur de la cuvette le long d'une paroi longitudinale (42) de celle-ci et présentant un bord inférieur (28) formant une arête de guidage et de retenue de la bille (46) précitée.

2. Cuvette selon la revendication 1, **caractérisée en ce que** la pièce de liaison (22) comporte des moyens d'accrochage sur la pièce de liaison d'une autre cuvette et d'articulation autour d'un axe transversal (xx') parallèle à un bord de l'extrémité supérieure de la cuvette.

3. Cuvette selon la revendication 2, **caractérisée en ce que** les moyens d'accrochage et d'articulation de la pièce de liaison (22) sont des moyens de clipsage ou d'encliquetage élastique.

4. Cuvette selon l'une des revendications 1 à 3, **caractérisée en ce que** la pièce de liaison comprend deux plaques minces ou lamelles (26) précitées qui sont parallèles et s'étendent vers le bas le long de deux parois longitudinales opposées de la cuvette et dont les bords inférieurs (28) forment deux arêtes parallèles de guidage et de retenue de la bille (46) précitée.

5. Cuvette selon la revendication 4, **caractérisée en ce que** les parties supérieures des plaques minces ou lamelles (26) précitées comportent une surépaisseur sur leur face tournée vers une grande paroi de la cuvette, cette surépaisseur formant un bourrelet d'étanchéité.

6. Cuvette selon la revendication4 ou 5, **caractérisée en ce que** les faces internes des grandes parois (42) de la cuvette ont une surface dépolie ou rugueuse, au moins en partie supérieure.

7. Cuvette selon l'une des revendications 1 à 4, **caractérisée en ce que** les bords verticaux de la plaque mince ou de chaque plaque mince ou lamelle (26) précitée sont guidés entre la paroi longitudinale (42, 52) de la cuvette et des nervures verticales (40) formées en saillie sur des faces internes des parois transversales (18) de la cuvette.

8. Cuvette selon l'une des revendications précédentes, **caractérisée en ce que** la pièce de liaison (22) comprend un cadre rectangulaire (24) qui coiffe l'extrémité supérieure de la cuvette et qui porte les moyens (32, 38) d'accrochage sur une pièce de liaison d'une autre cuvette.

9. Cuvette selon la revendication 8, **caractérisée en ce que** le cadre rectangulaire (24) de la pièce de liaison comprend deux bras parallèles (34) s'étendant vers l'extérieur dans le prolongement de ses petits côtés (30), les extrémités de ces deux bras comportant des moyens d'accrochage sur des moyens complémentaires formés sur le cadre rectangulaire (24) d'une pièce de liaison d'une autre cuvette.

10. Cuvette selon la revendication 8 ou 9, **caractérisée en ce que** les petits côtés du cadre rectangulaire (24, 64) comportent des rebords (31) de clipsage dans des fenêtres (32) des extrémités supérieures des petites parois de la cuvette (10).

11. Cuvette selon l'une des revendications précédentes, **caractérisée en ce que** la pièce de liaison (22) est en matériau plastique sensiblement opaque.

12. Ensemble de cuvettes de réaction pour appareil automatique d'analyse chimique ou biologique, **caractérisé en ce qu'**il comprend une pluralité de cuvettes de réaction (10) selon l'une des revendications précédentes, qui sont reliées les unes aux autres en file continue par les pièces de liaison (22) et qui sont enroulées en spirale sur un support circulaire de chargement dans l'appareil d'analyse ou disposées en rangées parallèles dans un chargeur vertical ou horizontal.

13. Ensemble comportant au moins une cuvette de réaction selon la revendication 8 et un dispositif de préhension de ladite cuvette de réaction, **caractérisée en ce que** le dispositif de préhension comprend une tige cylindrique (82) mobile en déplacement vertical et portant des moyens (86, 88) d'accrochage sur le cadre rectangulaire de la pièce de liaison, ces moyens d'accrochage étant déplaçables en translation horizontale sur la tige cylindrique (82).

14. Procédé d'assemblage de cuvettes de réaction selon l'une des revendications 1 à 11, comprenant les étapes consistant à :
- déposer des cuvettes de réaction sur des supports (98) comportant des emplacements de réception et de positionnement des cuvettes, ces emplacements étant agencés pour former des files de cuvettes espacées les unes des autres d'un pas prédéterminé ;
- déposer un moyen d'agitation, tel par exemple qu'une bille ferromagnétique (46), dans chacune des cuvettes positionnées sur les supports (98) précités ;
- déposer et encliqueter élastiquement des pièces de liaison sur les cuvettes positionnées sur les supports précités,
- vérifier par contrôle vidéo la présence des moyens d'agitation dans les cuvettes et l'encliquetage des pièces de liaison sur la cuvette, et
- déplacer les supports dans différents postes conçus pour effectuer les étapes précitées.

## Patentansprüche

1. Reaktionsküvette für ein automatisches chemisches oder biologisches Analysegerät, wobei diese Küvette ein oberes offenes Ende hat und ein Rührmittel für ein Fluid enthält, in der das obere Ende der Küvette (10) ein Verbindungsstück (22) zum Befestigen an einem identischen Verbindungsstück (22) einer anderen Küvette (10) gleichen Typs trägt, wobei das Verbindungsstück (22) durch Anclippen oder federelastisches Einrasten auf dem oberen Ende der Küvette befestigt wird, wobei das Verbindungsstück (22) Rückhaltemittel (28) für das Rührmittel des Fluids (46) umfasst, wobei sich diese Rückhaltemittel im Innern der Küvette erstrecken, wobei das Rührmittel für das Fluid eine Kugel (46) aus ferromagnetischen Material ist, und die auf dem Verbindungsstück (22) geformten Rückhaltemittel zumindest eine dünne Platte oder Lamelle (26) umfassen, die sich nach unten in das Innere der Küvette entlang einer Längswand (42) derselben erstreckt und einen Innenrand (28) aufweist, der eine Führungs- und Rückhaltekante für die vorgenannte Kugel (46) bildet.

2. Küvette nach Anspruch 1, **dadurch gekennzeichnet, dass** das Verbindungsstück (22) Mittel zum Befestigung am Verbindungsstück einer anderen Küvette und zur gelenkigen Verbindung um eine Querachse (XX') parallel zu einem Rand des oberen Endes der Küvette umfasst.

3. Küvette nach Anspruch 2, **dadurch gekennzeichnet, dass** die Befestigung- und Gelenkverbindungsmittel des Verbindungsstücks (22) Mittel zum Anclippen oder federelastischen Einrasten sind.

4. Küvette nach einem der Ansprüche 1 bis 3, **dadurch gekennzeichnet, dass** das Verbindungsstück zwei dünne Platten oder Lamellen (26) umfasst, die parallel zueinander stehen und sich nach unten entlang zweier gegenüber liegender Längswände der Küvette erstrecken und deren untere Ränder (28) zwei parallel verlaufende Führungs- und Rückhaltekanten für die vorgenannte Kugel (46) bilden.

5. Küvette nach Anspruch 4, **dadurch gekennzeichnet, dass** die oberen Teile der dünnen Platten oder Lamellen (26) eine Verdickung an ihrer zu einer großen Wand der Küvette gedrehten Fläche umfassen, wobei diese Verdickung einen Dichtwulst bildet.

6. Küvette nach Anspruch 4 oder 5, **dadurch gekennzeichnet, dass** die Innenflächen der großen Wände (42) der Küvette zumindest im oberen Teil eine matte oder raue Oberfläche haben.

7. Küvette nach einem der Ansprüche 1 bis 4, **dadurch gekennzeichnet, dass** die vertikalen Ränder der dünnen Platte oder jeder dünnen Platte oder Lamelle (26) zwischen der Längswand (42, 52) der Küvette und den vertikalen Rippen (40), die vor springend an den Innenflächen der Querwände (18) der Küvette gebildet sind, geführt werden.

8. Küvette nach einem der vorhergehenden Ansprüche, **dadurch gekennzeichnet, dass** das Verbindungsstück (22) einen rechteckigen Rahmen (24) umfasst, der das obere Ende der Küvette bedeckt und die Mittel (32, 38) zur Befestigung an einem Verbindungsstück einer anderen Küvette trägt.

9. Küvette nach Anspruch 8, **dadurch gekennzeichnet, dass** der rechteckige Rahmen (24) des Verbindungsstücks zwei parallele Arme (34) umfasst, die sich in der Verlängerung ihrer kleinen Seiten (30) nach außen erstrecken, wobei die Enden dieser beiden Arme Mittel zur Befestigung an den auf dem rechteckigen Rahmen (24) eines Verbindungsstücks einer anderen Küvette gebildeten komplementären Mittel umfasst.

10. Küvette nach Anspruch 8 oder 9, **dadurch gekennzeichnet, dass** die kleinen Seiten des rechteckigen Rahmens (24, 64) Clipkanten (31) in den Öffnungen (32) der oberen Enden der kleinen Wände der Küvette (10) umfassen.

11. Küvette nach einem der vorhergehenden Ansprüche, **dadurch gekennzeichnet, dass** das Verbindungsstück (22) aus im Wesentlichen opakem Kunststoff besteht.

12. Anordnung von Reaktionsküvetten für ein automatisches chemisches oder biologisches Analysegerät, **dadurch gekennzeichnet, dass** es eine Vielzahl an Küvetten (10) nach einem der vorhergehenden Ansprüche umfasst, die in einer durchgehenden Reihe durch Verbindungsstücke (22) miteinander verbunden sind und spiralförmig auf einer runden Halterung zum Einsetzten in das Analysegerät gewickelt oder in parallelen Reihen in einem vertikalen oder horizontalen Beschicker angeordnet sind.

13. Anordnung mit mindestens einer Reaktionsküvette nach Anspruch 8 und einer Vorrichtung zum Greifen dieser Reaktionsküvette, **dadurch gekennzeichnet, dass** die Greifvorrichtung einen vertikal beweglichen zylindrischen Schaft (82) umfasst, der die Mittel (86, 88) zur Befestigung am rechteckigen Rahmen des Verbindungsstücks trägt, wobei diese Befestigungsmittel in der Horizontalen translatorisch auf dem zylindrischen Schaft (82) verschiebbar sind.

14. Montageverfahren für Reaktionsküvetten nach einem der Ansprüche 1 bis 11, welches die folgenden Schritte enthält:
- das Einlegen der Reaktionsküvette auf Halterungen (98), welche Aufnahme- und Positionierungsstellen für die Küvetten umfassen, wobei diese Stellen so angeordnet sind, dass Küvettenreihen gebildet werden, in denen die Küvetten in einem vorab festgelegten Abstand zueinander stehen;
- das Einlegen eines Rührmittels, wie zum Beispiel einer ferromagnetischen Kugel (46), in jede der auf den Halterungen (98) positionieren Küvetten;
- das Einlegen und federelastische Einrasten der Verbindungsstücke auf den auf den Halterungen positionierten Küvetten,
- die Überprüfung des Vorhandenseins der Rührmittel in den Küvetten und des Einrastens der Verbindungsstücke auf der Küvette per Videokontrolle, und
- das Verschieben der Halterungen an verschiedene Stationen, die zur Durchführung dieser Schritte konzipiert sind.

## Claims

1. A reaction vessel for an automatic chemical or biological analysis device, with said vessel having an open upper end and containing a means for stirring a fluid, wherein the upper end of the vessel (10) carries a part (22) for connecting same, by snapping, with an identical connecting part (22) of another vessel (10) of the same type, with the connecting part (22) being clipped or elastically snap-fastened to the upper end of the vessel, with said connecting part (22) comprising means (28) for retaining the fluid stirring means (46), such retaining means extending inside the vessel, with the fluid stirring means being a ball (46) made of a ferromagnetic material, the retaining means formed on the connecting part (22) comprising at least one thin plate or lamella (26) extending downwardly inside the vessel along a longitudinal wall (42) thereof and having a lower edge (28) forming one edge guiding and retaining the ball (46) mentioned above.

2. A vessel according to claim 1, **characterized in that** the connecting part (22) comprises means for snapping onto the connecting part of another vessel and for providing rotation about a transversal axis (xx') parallel to one edge of the upper end of the vessel.

3. A vessel according to claim 2, **characterized in that** the means for snapping and rotating the connecting part (22) are clipping or elastically snap-fastening means.

4. A vessel according to one of claims 1 to 3, **characterized in that** the connecting part comprises two thin plates or lamellae (26) mentioned above which are parallel and extend downwardly along two opposite longitudinal walls of the vessel and the lower edges (28) of which form two parallel edges for guiding and retaining the ball (46) mentioned above.

5. A vessel according to claim 4, **characterized in that** the upper portions of the thin plates or lamellae (26) mentioned above include an extra thickness on the side thereof facing a large wall of the vessel, with said extra thickness forming a sealing bead.

6. A vessel according to claim 4 or 5, **characterized in that** the inner faces of the large walls (42) of the vessel have a roughened or rough surface at least in the upper part thereof.

7. A vessel according to claim 1 or 4, **characterized in that** the vertical edges of the thin plate or of each thin plate or lamella (26) mentioned above are guided between the longitudinal wall (42, 52) of the vessel and vertical ribs (40) formed so as to project from inner faces of the transverse walls (18) of the vessel.

8. A vessel according to one of the preceding claims, **characterized in that** the connecting part (22) comprises a rectangular frame (24) which covers the upper end of the vessel and which carries the means (32, 38) for snapping onto a connecting part of another vessel.

9. A vessel according to claim 8, **characterized in that** the rectangular frame (24) of the connecting part comprises two parallel arms (34) extending outwards as an extension of the smaller sides (30) thereof, with the ends of said two arms having means for snapping onto complementary means formed on the rectangular frame (24) of a connecting part of another vessel.

10. A vessel according to claim 8 or 9, **characterized in that** the smaller sides of the rectangular frame (24, 64) have flanges (31) snapping into windows (32) of the upper ends of the smaller walls of the vessel (10).

11. A vessel according to one of the preceding claims, **characterized in that** the connecting part (22) is made of a substantially opaque plastic material.

12. A set of reaction vessels for an automatic chemical or biological analysis device, **characterized in that** it comprises a plurality of reaction vessels (10) according to one of the preceding claims, which are connected to one another in a continuous line through the connecting parts (22) and which are spirally wound on a circular load support in the analysis device or arranged in parallel rows in a vertical or horizontal loader.

13. A set comprising at least one reaction vessel according to claim 8 and a device for gripping said reaction vessel, **characterized in that** the gripping device comprises a cylindrical rod (82) movable in vertical displacement and bearing means (86, 88) for snapping onto the rectangular frame of the connecting part, with said snapping means being movable in a horizontal translation on the cylindrical rod (82).

14. A method for assembling reaction vessels according to one of claims 1 to 11, comprising the steps of:
- depositing reaction vessels on supports (98) having recesses for receiving and positioning the vessels, with such recessing being so arranged as to form vessels rows spaced from each other by a predetermined step;
- depositing a stirring means, such as for example a ferromagnetic ball (46), into each of the vessels positioned on the supports (98) mentioned above;
- depositing and elastically snap-fastening connecting parts on the vessels positioned on the substrates mentioned above,
- checking, using video monitoring, the presence of the stirring means in the vessels and the snapping of the connecting parts on the vessel, and
- moving the supports to various stations designed to perform the steps mentioned above.
